(19) Europäisches Patentamt
European Patent Office
Office européen des brevets

(11) Publication number: **0 144 951**
**B1**

(12) **EUROPEAN PATENT SPECIFICATION**

(45) Date of publication of patent specification: **18.03.87**

(51) Int. Cl.$^4$: **C 07 C 87/24,** C 07 C 103/44, A 61 K 31/13, A 61 K 31/16

(21) Application number: **84114652.5**

(22) Date of filing: **03.12.84**

(54) **N-2,3-butadienyl-1,4-butanediamine derivatives.**

(30) Priority: **06.12.83 US 558642**

(43) Date of publication of application:
**19.06.85 Bulletin 85/25**

(45) Publication of the grant of the patent:
**18.03.87 Bulletin 87/12**

(84) Designated Contracting States:
**AT BE CH DE FR GB IT LI LU NL SE**

(56) References cited:
**US-A-3 712 896**

**CHEMICAL ABSTRACTS, vol. 92, no. 23, 9th June 1980, page 251, no. 193370b, Columbus, Ohio, US; A. KRANTZ et al.: "Inactivation of mitochondrial monoamine oxidase by beta,gamma,delta-allenic amines"**

(73) Proprietor: **MERRELL DOW PHARMACEUTICALS INC.**
**2110 E. Galbraith Road**
**Cincinnati Ohio 45215 (US)**

(72) Inventor: **Bey, Philippe**
**7875 Ivygate Lane**
**Cincinnati Ohio 45242 (US)**

(74) Representative: **Sgarbi, Renato et al**
**GRUPPO LEPETIT S.p.A. Patent & Trademark Dept. Via Giovanni Durando, 38**
**I-20158 Milano (IT)**

EP 0 144 951 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Description

Polyamine oxidase (PAO) is an enzyme which is present in high amounts in most mammalian tissues. PAO is responsible for the degradation of $N^1$-acetyl-spermidine and $N^1$-acetylspermine, to putrescine and spermidine, respectively [See F. Bolkenius *et al., Int. J. Biochem., 13,* 287 (1981)]. Hence, PAO is a key enzyme of the catabolic side of the polyamine interconversion pathway [See N. Seiler *et al., Medical Biology, 59,* 334 (1981)].

The present invention is directed to a novel class of chemical compounds whose structural formulae are represented by formula I below:

$$H_2C{=}C{=}CHCH_2NHCH_2{-}Z{-}CH_2NHR \qquad \text{Formula I}$$

wherein:

Z is $-CH_2CH_2-$ or *trans*-$CH{=}CH-$,

R is H, $CH_3-$, $CH_3CH_2$, $CH_3(CH_2)_2-$, $-(CH_2)_3NH_2$, $-(CH_2)_3NHCOCH_3$, $-CH_2CH{=}CH_2$, or $-CH_2CH{=}C{=}CH_2$, or a pharmaceutically acceptable acid addition salt thereof.

Examples of the compounds of Formula I are:

A. When Z is $-CH_2CH_2-$:

i R = H:
N-2,3-butadienyl-1,4-butanediamine

ii R = $CH_3-$, $CH_3CH_2-$, or $CH_3CH_2CH_2-$:
$N^1$-2,3-butadienyl-$N^4$-methyl-1,4-butanediamine
$N^1$-2,3-butadienyl-$N^4$-ethyl-1,4-butanediamine
$N^1$-2,3-butadienyl-$N^4$-propyl-1,4-butanediamine

iii R = $-CH_2CH{=}C{=}CH_2$:
$N^1$,$N^4$-2,3-butadienyl-1,4-butanediamine

iv R = $-CH_2CH{=}CH_2$:
$N^1$-2,3-butadienyl-$N^4$-1-propenyl-1,4-butanediamine

v R = $-(CH_2)_3NH_2$:
$N^1$-2,3-butadienyl-$N^4$-aminopropyl-1,4-butanediamine

vi R = $-(CH_2)_3NHCOCH_3$:
$N^1$-2,3-butadienyl-$N^4$-acetylaminopropyl-1,4-butanediamine

B. When Z is (*trans*)-$CH{=}CH-$:

vii R = H:
(E)-N-2,3-butadienyl-2-butene-1,4-diamine

viii R = $CH_3-$:
(E)-N-2,3-butadienyl-$N^4$-methyl-2-butene-1,4-diamine
(E)-N-2,3-butadienyl-$N^4$-ethyl-2-butene-1,4-diamine
(E)-N-2,3-butadienyl-$N^4$-propyl-2-butene-1,4-diamine

ix R = $-CH_2CH{=}C{=}CH_2$:
(E)-$N^1$,$N^4$-2,3-butadienyl-2-butane-1,4-diamine

x R = $-CH_2CH{=}CH_2$:
(E)-$N^1$-2,3-butadienyl-$N^4$-1-propenyl-2-butene-1,4-diamine

xi R = $-(CH_2)_3NH_2$:
(E)-$N^1$-2,3-butadienyl-$N^4$-aminopropyl-2-butene-1,4-diamine

xii R = $-(CH_2)_3NHCOCH_3$:
(E)-$N^1$-2,3-butadienyl-$N^4$-acetylaminopropyl-2-butene-1,4-diamine

Illustrative examples of pharmaceutically acceptable salts of the compounds of this invention include non-toxic acid addition salts formed with inorganic acids, such as hydrochloric, hydrobromic, sulfuric and phosphoric acid, or with organic acids, such as organic carboxylic acids, for example salicylic, maleic, malonic, tartaric, citric and ascorbic acids and organic sulfonic acids, for example methane sulfonic acid.

The compounds of Formula I are irreversible inhibitors of polyamine oxidase (PAO) as can be demonstrated *in vitro* and *in vivo* in biochemical test procedures. The biochemical testing of illustrative compounds for their ability to inhibit PAO is illustrated herein in Examples 7 and 8.

Inhibitors of PAO are of particular interest for the study of the physiological role of the polyamine interconversion pathways in mammals. Additionally, inhibitors of PAO prevent the degradation of $N^1$-acetylspermidine with the concomitant formation of putrescine. The ability to decrease the amount of circulating putrescine in mammals would be highly advantageous with certain conditions, such as for example, in situations of enhanced cell proliferation.

It is believed that the compounds of Formula I are "substrate-induced irreversible inhibitors" of PAO. Such inhibitors are also known in the art as "enzyme-activated irreversible inhibitors", "suicide enzyme inhibitors", "$K_{cat}$ inhibitors", or "mechanism-based inhibitors". In order for a compound to be a substrate-induced irreversible enzyme inhibitor, the compound must be a substrate for the *target* enzyme, and the compound must contain a latent reactive group susceptible to being unmasked as the result of the normal catalytic action of the enzyme. The unmasking of the latent reactive group by the action of the enzyme

generates a reactive function which alkylates a nucleophilic residue present at the active site of the enzyme. Thus, there is formed a covalent bond between the inhibitor and the enzyme at the active site resulting in irreversible inactivation of the enzyme. Such inhibitors are extremely specific since the inhibitor must be a substrate for the target enzyme and since biotransformation of the inhibitor by the target enzyme is required before the enzyme is inactivated. Although it is believed that the compounds of Formula I generally exert their action by means of a substrate-induced mechanism, inhibition may occur by other mechanisms, such as by competitive inhibition.

In general, the compounds of Formula I can be prepared by treating a compound Formula II:

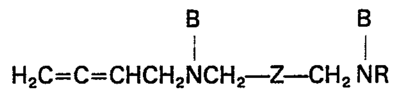

Formula II

wherein:

Z is $-CH_2-CH_2-$ or (*trans*)-$CH=CH-$,

B is an amino-protecting group, and

R is $-H$, $-CH_3$, $-CH_2CH_3$, $-CH_2CH_2CH_3$, $-(CH_2)_3NH_2$, $-(CH_2)_3NHCOCH_3$, $-CH_2CH=CH_2$, or $-CH_2CH=C=CH_2$;

in a known manner to be useful for removing the aminoprotecting groups (B), with the proviso that when R is $-(CH_2)_3NHCOCH_3$, the amino-protecting group (B) must be tertiobutyloxycarbonyl.

Examples of compounds of Formula II are:

A. Wherein Z is $-CH_2CH_2-$ and B is tertiobutyloxycarbonyl:

xiii R is H:
$N^1,N^4$-tertiobutyloxycarbonyl-$N^1$-2,3-butadienyl-1,4-butanediamine

xiv R is methyl, ethyl, or propyl:
$N^1,N^4$-tertiobutyloxycarbonyl-$N^1$-2,3-butadienyl-$N^4$-methyl-1,4-butanediamine
$N^1,N^4$-tertiobutyloxycarbonyl-$N^1$-2,3-butadienyl-$N^4$-ethyl-1,4-butanediamine
$N^1,N^4$-tertiobutyloxycarbonyl-$N^1$-2,3-butadienyl-$N^4$-propyl-1,4-butanediamine

xv R is $-CH_2C=C=CH_2$:
$N^1,N^4$-tertiobutyloxycarbonyl-$N^1,N^4$-2,3-butadienyl-1,4-butanediamine

xvi R is $-CH_2-CH=CH_2$:
$N^1,N^4$-tertiobutyloxycarbonyl-$N^1$-2,3-butadienyl-$N^4$-1-propenyl-1,4-butanediamine

xvii R is $-(CH_2)_3NH_2$:
$N^1,N^4$-tertiobutyloxycarbonyl-$N^1$-2,3-butadienyl-$N^4$-aminopropyl-1,4-butanediamine

xviii R is $-(CH_2)_3NHCOCH_3$:
$N^1,N^4$-tertiobutyloxycarbonyl-$N^1$-2,3-butadienyl-$N^4$-acetylaminopropyl-1,4-butanediamine

B. Wherein Z is *trans*-$CH=CH_2-$:

xix R is H:
(E)-$N^1,N^4$-tertiobutyloxycarbonyl-$N^1$-2,3-butadienyl-2-butene-1,4-diamine

xx R is $CH_3-$, $CH_3CH_2-$, or $CH_3CH_2CH_2-$:
(E)-$N^1,N^4$-tertiobutyloxycarbonyl-$N^1$-2,3-butadienyl-$N^4$-methyl-2-butene-1,4-diamine
(E)-$N^1,N^4$-tertiobutyloxycarbonyl-$N^1$-2,3-butadienyl-$N^4$-ethyl-2-butene-1,4-diamine
(E)-$N^1,N^4$-tertiobutyloxycarbonyl-$N^1$-2,3-butadienyl-$N^4$-propyl-2-butene-1,4-diamine

xxi R is $-CH_2CH=C=CH_2$:
(E)-$N^1,N^4$-tertiobutyloxycarbonyl-$N^1,N^4$-2,3-butadienyl-2-butene-1,4-diamine

xxii R is $-CH_2CH=CH_2$:
(E)-$N^1,N^4$-tertiobutyloxycarbonyl-$N^1$-2,3-butadienyl-$N^4$-1-propenyl-2-butene-1,4-diamine

xxiii R is $-(CH_2)_3NH_2$:
(E)-$N^1,N^4$-tertiobutyloxycarbonyl-$N^1$-2,3-butadienyl-$N^4$-aminopropyl-2-butene-1,4-diamine

xxiv R is $-(CH_2)_3NHCOCH_3$:
(E)-$N^1,N^4$-tertiobutyloxycarbonyl-$N^1$-2,3-butadienyl-$N^4$-acetylaminopropyl-2-butene-1,4-diamine.

The amino-protecting groups (B) are chosen with regard to the nature of the relevant reactions used to prepare the particular compounds of Formula II and having regard to the ease of their removal. The protecting groups include lower alkanoyl, e.g. acetyl, propionyl and trifluoroacetyl; aroyl, e.g. benzoyl, toluoyl; lower alkoxycarbonyl, for example, methoxycarbonyl, ethoxycarbonyl and tertiobutoxycarbonyl; carbobenzoxy; benzenesulfonyl; and tosyl.

In the preparation of the compounds of Formula II, the protecting groups are introduced in a known manner such as the reaction of an appropriate primary or secondary amine with a lower alkanoyl or aroyl chloride, anhydride, sulfonyl-chloride, tertiobutoxycarbonyloxyimino-2-phenyl-acetonitrile (BOC—ON), or di-tertiobutyl dicarbonate [$(BOC)_2O$]. A preferred amino-protecting group is tertiobutoxycarbonyl (BOC).

Removal of the protecting groups from the compounds of Formula II is conducted in a manner known to those skilled in the art for the relevant protecting group. Usually, said removal involves hydrolytic cleavage using an organic or mineral acid such as trifluoroacetic acid, hydrochloric acid and the like; or by hydrogen chloride gas under anhydrous conditions. Solvents used will be chosen dependent upon the conditions of protecting group removal. For example, ethers such as diethylether can be used for hydrolytic cleavage with hydrogen chloride gas. If other acid sensitive functional groups are present in the molecule,

the acid conditions chosen for the removal of the protecting group must be mild in order to avoid unwanted side reactions. In the case of a carbobenzoxy protecting group, this group can be removed in a known manner via catalytic hydrogenolysis.

The compounds of Formula II wherein Z is $—CH_2CH_2—$ or (*trans*)-CH=CH—, R is hydrogen and B is tertiobutyloxycarbonyl (BOC) are prepared by the method depicted below in Scheme I starting with a N-tertiobutyloxycarbonyl-γ-aminobutyric acid or N-tertiobutyloxycarbonyl-γ-amino-α,β-dihydrobutyric acid as depicted in Formula III:

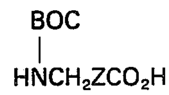

Formula III

wherein Z is $—CH_2CH_2—$ or (*trans*)-CH=CH—.

SCHEME I

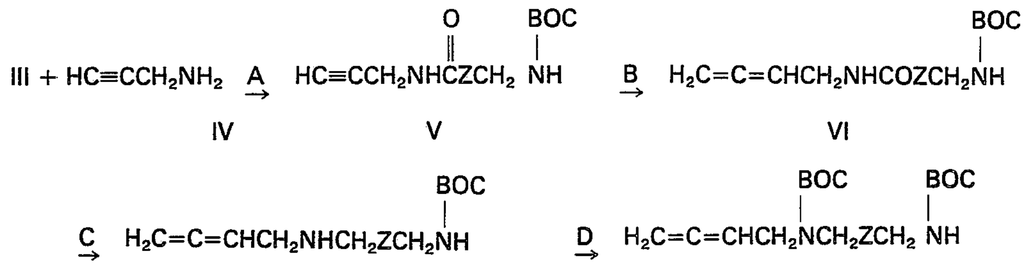


IV V VI

VII VIII

In *Step A* of *Scheme I,* a compound of Formula III is reacted in a known manner with propargylamine (IV) to yield a compound of Formula V. The reaction is conveniently performed in the presence of N,N[1]-dicyclohexylcarbodiimide in an organic solvent, for example acetonitrile. The ethynyl group of Compound V can be converted to the allenyl group of Compound VI in a known manner using the general method described by P. Crabbé *et al., J.C.S. Chem. Comm.* 859—860 (1979) and H. Fillion *et al., Tet. Letters,* 929—930 (1980) for allenic alcohols. In accordance with this procedure the amino protected derivative of a compound of Formula V is treated with formaldehyde and a secondary amine having a hydrogen atom on the α-carbon atom and heated in an organic solvent in the presence of an inorganic salt. Preferably, the heating utilizes reflux conditions. The preferred amine is diisopropylamine and the preferred inorganic salt is a copper salt, particularly cuprous bromide or cupric chloride. Suitable solvents include dioxane, tetrahydrofuran, 1,2-dimethoxyethane, benzene, acetonitrile and/or toluene. The conversion is deemed to proceed via the corresponding amino protected derivative of the secondary amino propynyl compound.

Compound VI is selectively reduced in *Step C* in a known manner to a compound of Formula VII. The reduction of the carbonyl group is conveniently achieved by means of lithium aluminium hydride in diethyl ether. Compound VII can thus be converted in a known manner to a compound of Formula VIII utilizing conventional procedures for the introduction of a BOC protecting group on a secondary amine. Thus, for example, the secondary amine can be protected by treatment with ditertiobutyl-carbonate in tetrahydrofuran (THF) at the reflux temperature.

The preparation of $N,N^4$-tertiobutyloxycarbonyl-$N^1$-2,3-butadienyl-1,4-butanediamine from 4-tertiobutyloxycarbonylamino-γ-butyric acid according to the general method of Scheme I is specifically described in Example 1.

In general, the compounds of Formula II wherein Z is $—CH_2CH_2—$ or (*trans*)-CH=CH— and R is $—CH_3$, $—CH_2CH_3$, or $—CH_2CH_2CH_3$, $—CH_2CH=C=CH_2$, or $—CH_2CH=CH_2$ are prepared in a known manner by the reaction of an N-protected-2,3-butadienylamine of Formula IX:

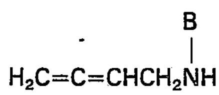

Formula IX

wherein B is an amino-protecting group, with a compound of formula X:

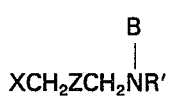

Formula X

wherein Z is $—CH_2CH_2—$ or (*trans*)-CH=CH—, B is an amino-protecting group, R′ is $—CH_3$, $—CH_2CH_3$, or $—CH_2CH_2CH_3$, $—CH_2CH=C=CH_2$, or $—CH_2CH=CH_2$, and X is a leaving group. Preferred leaving groups are:

mesylate, tosylate, bromide, or iodide. Iodide is the most preferred leaving group. The reaction can conveniently be carried out in an organic solvent, such as tetrahydrofuran (THF), diethyl ether, dimethylformamide (DMF), or benzene, in the presence of one equivalent of strong base, such as, potassium or sodium hydride, potassium or sodium *tert*-butoxide, or lithium diisopropylamide, for a period ranging from 10 minutes to 24 hours at −30°C to 100°C, optimally in the presence of a catalytic amount of sodium iodide. The preferred reaction conditions utilize sodium hydride in DMF at 0 to 25°C. The preparation of $N^1$,$N^4$-tertiobutyl-oxycarbonyl-$N^1$-2,3-butadienyl-$N^4$-methyl-1,4-butanediamine from N-tertiobutyloxycarbonyl-2,3-butadienylamine (IX) and N-tertiobutyloxycarbonyl-N-methyl-4-iodo-1-butenamine (X) is specifically described in Example 4a.

The compounds of Formula II wherein Z is $—CH_2CH_2—$ or (*trans*)-CH=CH— and R is $—CH_2CH=C=CH_2$, can also be prepared by the reaction of two equivalents of a compound of Formula IX with a compound of Formula XI:

$$XCH_2ZCH_2X$$ Formula XI

wherein Z is $—CH_2CH_2—$ or (*trans*)-CH=CH— and X is a leaving group, such as those defined with respect to Formula X. This reaction can be conducted in a known manner as previously described with respect to the reaction of a compound of Formula IX with a compound of Formula X. The preparation of $N^1$,$N^4$-tertiobutyloxycarbonyl-$N^1$,$N^4$-2,3-butadienyl-1,4-butanediamine via N-tertiobutyloxycarbonyl-2,3-butadienylamine (IX) and 1,4-diiodobutane (XI) is specifically described in Example 5a. The preparation of (E)-$N^1$,$N^4$-tertiobutyloxycarbonyl-$N^1$,$N^4$-2,3-butadienyl-2-butane-1,4-diamine from N-tertiobutyloxycarbonyl-2,3-butadienylamine and (E)-1,4-dibromo-2-butene is described in Example 6A.

The compounds of formula II wherein Z is $—CH_2CH_2—$ or (*trans*)-CH=CH— and R is $—(CH_2)_3NHCOCH_3$ or $—(CH_2)_3NH_2$ are made in manner known *per se* by the reaction sequence depicted below in Scheme II starting from a compound of Formula VIII (see Scheme I):

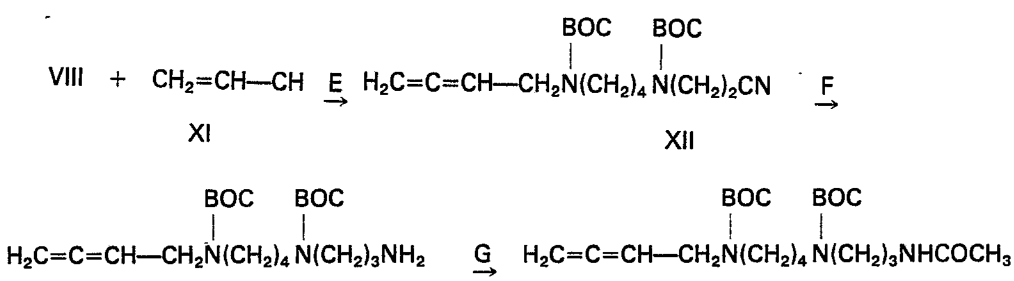


XI XII XIII XIV

In *Step E,* a compound of Formula VIII is reacted with acrylonitrile (XI) in a known manner to provide the compound of Formula XII. The reaction is performed in the presence of a base in an organic solvent.

In *Step F,* compound XII is reduced to yield the compound of Formula XIII in a known manner utilizing conventional (non-catalytic) methods for the selective reduction of a cyano group to a primary amino group. The preferred reagent is lithium aluminium hydride. The preparation of the N-acetyl derivative of Formula XIV from Compound XIII is carried out in a manner known to those skilled in the art using conventional acetylation techniques.

The N-protected 2,3-butadienyl amine compounds of Formula IX can be prepared in a known manner from an N-protected propargyl amine of Formula XV:

$$HC{\equiv}CCH_2NHB$$ Formula XV

wherein B is an amino-protecting group as previously defined. The procedure for carrying out this transformation is described above with respect to *Step B* in Scheme I. The specific preparation of N-tertiobutyloxycarbonyl-2,3-butadienylamine from N-tertiobutyloxypropargylamine is described in Example 3.

The compounds of Formula X can be prepared in a known manner utilizing an N-protected-γ-aminobutyric acid or N-protected-γ-amino-α,β-dihydro-butyric acid of Formula IIIa:

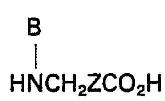

Formula IIIa

wherein B is an amino-protecting group and Z is $—CH_2CH_2$ or (*trans*)-CH=CH—, using the method depicted below in Scheme III or Scheme IV.

Scheme III is preferred for the preparation of compounds of Formula X wherein R is $—CH_3$, $—CH_2CH_3$, or $—CH_2CH_2CH_3$ (the compounds of general Formula XVIII, below),

SCHEME III

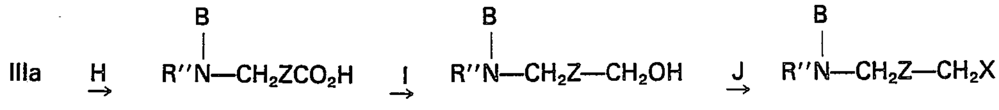


XVI XVII XVIII

In Scheme III, the symbol B is an amino-protecting group, R″ is —$CH_3$, —$CH_2CH_3$, or —$CH_2CH_2CH_3$, and X is a leaving group. In *Step H,* a compound of Formula IIIa is alkylated in a known manner to yield a compound of Formula XVI. The alkylation can be conducted by the reaction of a compound of Formula IIIa with 2 equivalents of a strong base, such as sodium hydride, in an aprotic solvent, such as THF, and then treating the anion thus formed with a suitable alkylating agent (RX) wherein X is a leaving group, preferably iodide.

Compound XVI is reduced to the corresponding alcohol of Formula XVII in *Step I* in known procedures utilizing a reagent capable of reducing the carboxylic acid function without affecting the N-protecting group. A suitable reagent for this reduction is diborane.

In *Step J,* an alcohol of Formula XVII is converted to a compound of Formula XVIII using conventional methods suitable for the replacement of an —OH group by a leaving group. The preparation of N-tertiobutyloxycarbonyl-N-methyl-4-iodo-1-butanamine (X) from 4-tertiobutyloxycarbonylamine-1-butyric acid (IIIa) is further described in Examples 2a, 2b, 2c, and 2d.

Scheme IV is preferred for the preparation of compounds of Formula X wherein R is —$CH_2CH{=}C{=}CH_2$ or —$CH_2CH{=}CH_2$ (the compounds of Formula XXI).

SCHEME IV

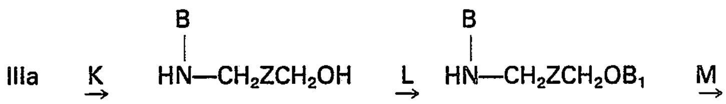


XIX XX

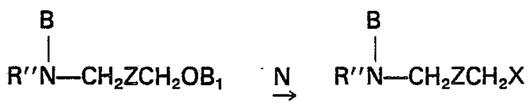


XXI XXII

In Scheme IV, R″ is —$CH_2CH{=}CH_2$ or —$CH_2CH{=}C{=}CH_2$, $B_1$ is a protecting group for an OH group. In *Step K,* a compound of Formula IIIa is selectively reduced in a known manner to yield the alcohol of Formula XIX. A suitable method for the reaction is described in *Step I* of Scheme III.

The OH group of compound XIX is thus protected in *Step L* via known procedures to yield a compound of Formula XX. Suitable protecting groups for the OH group are those that can be removed under mild acidic conditions, for example tetrahydropyranyl or methoxymethyl.

Compound XX is alkylated in *Step M* via known procedures to yield a compound of Formula XXI. A suitable procedure is described in Step H of Scheme III above, with the proviso that only one equivalent of base is employed.

In the final reaction, *Step N,* compound XXI is converted to a compound of Formula XXII via known methods useful in the replacement of an —OH group by a leaving group (X).

The following Examples further illustrate the invention and are not intended to limit the invention in any way:

## Example 1

## N-2,3-Butadienyl-1,4-butanediamine

A. 4-Tertiobutyloxycarbonylamino-N-(2-propynyl) butanamide

To a solution of 4-tertiobutyloxycarbonyl aminobutyric acid (5.05 g, 25 mM) and propargylamine (1.71 ml, 25 mM) in acetonitrile (120 ml) cooled to 0°C was added under nitrogen a solution of N,N$^1$-dicyclohexylcarbodiimide (5.15 g, 25 mM) in acetonitrile (20 ml). The temperature of the reaction mixture was slowly raised to 20°C, and stirring was continued for 12 h. Concentration *in vacuo* left a solid residue which was chromatographed on silica gel (400 g). Elution with a gradient of ethyl acetate/dichloromethane (1/5 to 2/5) gave the title compound after recrystallization from ethylacetate; mp 147°C.

B. 4-Tertiobutyloxycarbonylamino-N-(2,3-butadienyl) butanamide

A mixture of 4-tertiobutyloxycarbonylamino-N-(2-propynyl) butanamide (2.4 g, 10 mM), prepared as in Part A, diisopropylamine (1.68 ml, 12 mM), cuprous bromide (0.474 g, 3.3 mM), formaldehyde (1.19 ml of a

37% solution in water, 16 mM) in dioxane (20 ml) was treated at reflux temperature for 1 h. The reaction mixture was then quenched with water and extracted with methylene chloride (3 × 40 ml). The organic layers were pooled and washed with 1 N acetic acid, water, and brine, and dried over magnesium sulfate ($MgSO_4$). Concentration *in vacuo* left a solid residue (2.4 g) which was chromatographed on silica gel (150 g). Elution with ethyl acetate/chloroform (1/5) yielded the title compound after recrystallization in dichloromethane/pentane; mp 92°C.

C. $N^1$,$N^4$-Tertiobutyloxycarbonyl-$N^1$-2,3-butadienyl-1,4-butanediamine

To a suspension of lithium aluminium hydride (1.19 g, 31 mM) in ahydrous diethyl ether (150 ml) cooled to 0°C was added a solution of 4-tertiobutyloxycarbonylamino-N-(2,3-butadienyl)butanamide, prepared as in Part B (1.59 g. 6.3 mM) in anhydrous tetrahydrofuran (THF, 6 ml). After stirring for 54 h at room temperature, the reaction mixture was quenched successively with water (1.19 ml), 15% aqueous sodium hydroxide (1.19 ml), and water (3.57 ml). The aluminium salts were filtered and washéd extensively with diethyl ether. The filtrate was concentrated *in vacuo* to afford $N^4$-tertiobutyloxycarbonyl-$N^1$-2,3-butadienyl-1,4-butanediamine contaminated with a small amount of $N^1$-2,3-butadienyl-$N^4$-methyl-1,4-butanediamine as an oily residue (955 mg). This residue was dissolved in THF (40 ml). Ditertio-butyloxy-carbonate (0.9 g, 4.15 mM) was added, and the mixture was heated at reflux temperature for 4 h. Diethyl ether (100 ml) was added, and the organic phase was washed with water (3 × 20 ml), dried over $MgSO_4$, and concentrated *in vacuo.* The residue was purified by flash chromatography on silica gel (100 g). Elution with diethyl ether/petroleum ether (1/9) afforded $N^1$,$N^4$-tertiobutyloxycarbonyl-$N^1$-2,3-butadienyl-$N^4$-methyl-1,4-butanediamine (165 mg), then a mixture of $N^1$,$N^4$-tertiobutyloxycarbonyl-$N^1$-(2,3-butadienyl)-1,4-butanediamine and the former compound (344 mg), and finally pure $N^1$,$N^4$-tertiobutyloxycarbonyl-$N^1$-(2,3-butadienyl)-1,4-butanediamine (365 mg) as an oil:

IR ($CH_2Cl_2$): 1950 $cm^{-1}$ (allene), 1680 $cm^{-1}$, 1700 $cm^{-1}$ (—CO—);

NMR ($CDCl_3$): 5 ppm 1.46 ($C(CH_3)_3$, 18 H, s), 2.9—3.4 ($CH_2$—N, 4 H, m), 3.6—4.0 (=—$CH_2$—N, 2 H, m), 4.4—5.4

( 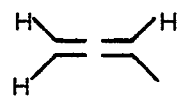 , 3 H, m).

D. N-2,3-Butadienyl-1,4-butanediamine

To a solution of $N^1$,$N^4$-tertiobutyloxycarbonyl-$N^1$-2-3-butadienyl-1,4-butanediamine, prepared as in Part C (0.3 g), in absolute ethanol (4 ml) was added a solution of anhydrous HCl (3.7 M) in diethyl ether (4 ml). The mixture was left standing 4 h at room temperature and 20 h at 0°C. The crystals which formed slowly were filtered and recrystallized from absolute ethanol to give the title compound, as the dihydrochloride (170 mg): mp 197°C;

NMR ($D_2O$) δ ppm: 1.8—2.0 (—$CH_2$—, 4 H, m), 2.8—3.4 ($CH_2$—N, 4 H, m), 3.5—3.8 (=—$CH_2$—N, 2 H, m), 5.0—5.6

( 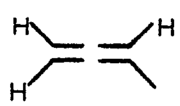 , 3 H, m).

## Example 2

## N-Tertiobutyloxycarbonyl-N-methyl-4-iodobutanamine

A. 4-Tertiobutyloxycarbonylamino-1-butyric acid

A mixture of GABA (51.5 g) and ditertiobutylcarbonate (109.12 g) in THF (100 ml) and water (100 ml) was heated under reflux temperature for 2 h. The solvents were concentrated *in vacuo* and the residue extracted with ether. The organic layers were pooled, washed with brine, and dried over $MgSO_4$. Concentration *in vacuo* gave the title compound (87.8 g); mp 51°C, recrystallized from pentane/$Et_2O$ at −80°C;

NMR ($CDCl_3$) δ: 1.45 ($C(CH_3)_3$, 9 H, s).

B. N-Tertiobutyloxycarbonyl-N-methyl-4-amino-1-butyric acid

To a suspension of sodium hydride (9.6 g of a 55% suspension in oil washed 3 times in pentane) in anhydrous THF (250 ml) was added a solution of 4-tertiobutyloxycarbonylamino-1-butyric acid, prepared as in Part A (20.3 g), in anhydrous THF (250 ml). The mixture was stirred for 20 h at 25°C and then methyl iodide (13.64 ml) was added. After 3 h at 25°C, the reaction mixture was quenched with brine, neutralized with acetic acid and extracted with diethyl ether many times. The organic layers were pooled, washed with brine, and dried over $MgSO_4$. Concentration *in vacuo* left an oily residue which was crystallized from diethyl ether/pentane to afford the title compound (17 g); mp 61°C;

NMR ($CDCl_3$): 1.43 ($C(CH_3)_3$, s, 9 H), 2.83 (N—$CH_3$, s, 3 H).

C. N-Tertiobutyloxycarbonyl-N-methyl-4-hydroxy-1-butanamine

To a solution of N-tertiobutyloxycarbonyl-N-methyl-4-amino-1-butyric acid, prepared as in Part C (10.08 g) in anhydrous THF (100 ml) cooled to −78°C was added under nitrogen a solution of borane (1 M) in THF (75 ml). The temperature was allowed to rise to 25°C and stirring was continued for 20 h at 25°C whereupon the excess of borane was destroyed by addition of methanol. Concentrated acetic acid was then added until the pH of the solution became acidic. The solvent was concentrated *in vacuo* and the residue extracted with diethyl ether. The organic layer was washed with water, an aqueous solution of bicarbonate, and then brine, dried over $MgSO_4$, and then concentrated *in vacuo* to yield the title compound (9.08 g) as an oil, which was used in the next step without further purification.

NMR ($CDCl_3$): 1.46 ($C(CH_3)_3$, s), 2.83 (N—$CH_3$, s, 3 H).

D. N-Tertiobutyloxycarbonyl-N-methyl-4-iodo-1-butanamine

To a solution of N-tertiobutyloxycarbonyl-N-methyl-4-hydroxy-1-butanamine, prepared as in Part C (4.84 g), and triethylamine (5 ml) in methylene chloride (75 ml) cooled to −10°C was added dropwise a solution of mesylchloride (3 g) in methylene chloride (25 ml). Stirring was continued for 15 min at −10°C. The reaction mixture was then quenched with water. The organic layer was washed consecutively with aqueous acetic acid (1 N), aqueous sodium bicarbonate, water, and brine, dried over $MgSO_4$, and concentrated *in vacuo* to afford the corresponding mesylate (6.29 g, 0.022 mol) as an oil.

NMR ($CDCl_3$): 1.46 ($C(CH_3)_3$, s), 2.83 (N—$CH_3$, s, 3H), 3.0 ($CH_3OSO_2$, s, 3 H).

The mesylate (6.29 g) was dissolved in anyhydrous diethyl ether (75 ml). The solution was cooled to −10°C. Then a solution of 0.15 M magnesium iodide (150 ml) in anhydrous ether was added under vigorous stirring. The reaction mixture was quenched with water. The etheral layer was washed with water, aqueous bisulfite and brine, dried over $MgSO_4$, and concentrated *in vacuo* to yield the title compound (6.25 g) as an oil.

NMR ($CDCl_3$): 1.46 ($C(CH_3)_3$, s), 2.8 (N—$CH_3$, 3 H, s).

Example 3

N-Tertiobutyloxycarbonyl-2,3-butadienylamine

A mixture of N-tertiobutyloxycarbonyl propargylamine (14.77 g, 0.0953 mol), diisopropylamine (19.97 ml, 0.114 mol), formaldehyde (19.58 ml of a 37% aqueous solution, 0.213 mol), and CuBr (4.1 g, 0.029 mol) in dioxane (180 ml) was heated at reflux temperature for 12 h under nitrogen. Upon cooling the solution was diluted with diethyl ether (~500 ml). Then acetic acid was added until the pH of the solution reached the value of 4. The organic layer was washed many times with water and then brine, dried over $MgSO_4$, and concentrated *in vacuo* to yield a brown oil (13 g) which was purified by flash chromatography (300 g of silica gel; eluant: diethyl ether/petroleum ether 1/9) to afford the title compound (10.14 g) as an oil.

TLC: Rf = 0.66 ($Et_2O$/Pet. ether 1/4);

NMR ($CDCl_3$): 1.45 ($C(CH_3)_3$, 9 H, s), 3.46—3.94 (—$CH_2$—N, 2 H, m), 4.5—5.4

( 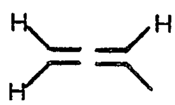 , and NH, 4 H, m).

Example 4

$N^1$-2,3-Butadienyl-$N^4$-methyl-1,4-butanediamine

A. $N^1$,$N^4$-Tertiobutyloxycarbonyl-$N^1$-2,3-butadienyl-$N^4$-methyl-1,4-butanediamine

To a suspension of sodium hydride (0.87 g of a 55% suspension in oil washed 3 times with pentane, 0.02 mmol) in anhydrous dimethylformamide (30 ml) cooled to 0°C was added under nitrogen a solution of N-tertiobutyloxycarbonyl-N-methyl-4-iodobutanamine, prepared as in Example 2 (6.25 g, 0.02 mol), in anhydrous dimethylformamide (20 ml). Then a solution of N-tertiobutyloxycarbonyl-2,3-butadienylamine prepared as in Example 3 (3.38 g, 0.02 mol) in anhydrous dimethylformamide (30 ml) was added over a period of 20 min. Stirring was continued for $2\frac{1}{2}$ h at 0°C. Then the reaction mixture was quenched with water and extracted with diethyl ether (5 × 100 ml). The organic layers were pooled, washed with water and then brine, and dried over $MgSO_4$. Concentration *in vacuo* gave an oil residue (8.3 g) which was purified by flash chromatography (400 g silica gel; eluant: diethyl ether/petroleum ether, 1/4) to afford the title compound (5.5 g) as an oil

TLC: Rf = 0.3 (diethyl ether/petroleum ether — 25/75);

NMR ($CDCl_3$): δ 1.45 ($C(CH_3)_3$ and —$CH_2CH_2$—, 22 H), 2.83 (N—$CH_3$, 3 H, s), 4.6—5.4

( 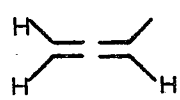 , 3 H, m).

IR ($CH_2Cl_2$): 1950 $cm^{-1}$

(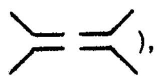),

1675 $cm^{-1}$ ($HNCO_2$—);

MS (CI, $CH_4$): m/e 355.

Analysis for $C_{19}H_{34}N_2O_4$:

Calc. C, 64.38; H, 9.68; N, 7.90%

Found C, 63.92; H, 9.48; N, 7.39%

The analytical sample was prepared by distillation with a "cold finger" apparatus (temperature oil bath 130°C, 0.3 mm Hg).

B. $N^1$-2,3-Butadienyl-$N^4$-methyl-1,4-butanediamine

To a solution of $N^1$,$N^4$-tertiobutyloxycarbonyl-$N^1$-2,3-butadienyl-$N^4$-methyl-1,4-butanediamine, prepared as in Part A (4.9 g), in absolute ethanol (25 ml) was added a solution of anhydrous HCl (2 M) in diethyl ether (80 ml). The mixture was left standing for 4 h at 25°C and then for 20 h at 4°C. The crystals which formed slowly were filtered and washed with anhydrous diethyl ether to yield the title compound as the dihydrochloride, analytically pure (2.86 g); mp 226°C.

NMR ($D_2O$: δ 280 (N—$CH_3$, 3 H, s),

5—5.8 ( 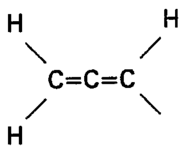 , m, 3 H);

IR (KBr): 1950 $cm^{-1}$

( 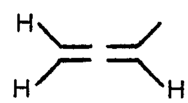 ).

Example 5

$N^1$,$N^4$-2,3-Butadienyl-1,4-butanediamine

A. $N^1$,$N^4$-Tertiobutyloxycarbonyl-$N^1$,$N^4$-2,3-butadienyl-1,4-butanediamine

To a suspension of sodium hydride (2.4 g of a 55% oil suspension washed 3 times with pentane, 0.055 mol) in anhydrous dimethylformamide (50 ml) cooled to 0°C, was added under nitrogen, a solution of 1,4-diiodobutane (7.75 g, 0.025 mol) in anhydrous dimethylformamide (10 ml). Then a solution of N-tertiobutyloxycarbonyl-2,3-butadienylamine, prepared as in Example 3 (8.45 g, 0.05 mol) in anhydrous dimethylformamide (40 ml) was added over a period of 25 min. Stirring was continued for 3 h at 25°C. The reaction mixture was then quenched with water and extracted with ether (5 × 150 ml). The organic layers were pooled, washed with water, and then brine, and dried over $MgSO_4$. Concentration *in vacuo* gave an oily residue (8.88 g) which was purified by flash chromatography (300 g silica gel; eluant: gradient of diethyl ether/petroleum ether from 5/95 to 20/80) to afford a complex mixture of unidentified compounds (4.4 g) and the title compound (4.11 g) as an oil.

TLC: Rf = 0.3 (eluant: diethyl ether/petroleum ether 25:75);

NMR ($CDCl_3$) δ 4.6—5.3

( 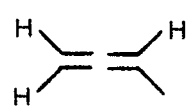 , 6 H, m);

1.45 ($C(CH_3)_3$, —$CH_2$—, 22 H, s);

IR ($CH_2Cl_2$): 1950 $cm^{-1}$ (=·=), 1675 $cm^{-1}$ (—$NCO_2$—);

MS (CI, $CH_4$) m/e $MH^+$ 393.

Analysis for $C_{22}H_{36}N_2O_4$:

Calc. C, 67.32: H, 9.24; N, 7.14%

Found C, 67.76; H, 9.12; N, 6.63%

The analytical sample was prepared by distillation with a "cold finger" apparatus (temp. oil bath 95°C, 0.01 mm Hg).

B. $N^1$,$N^4$-2,3-Butadienyl-1,4-butanediamine

To a solution of $N^1$,$N^4$-tertiobutyloxycarbonyl-$N^1$,$N^4$-2,3-butadienyl-1,4-butanediamine, prepared as in

Part A (4.1 g), in absolute ethanol (25 ml) was added a solution of anhydrous HCl (2 M) in anhydrous diethylether (80 ml). The mixture was left standing for 4 h at 25°C and then for 20 h at 4°C. The crystals which formed slowly were filtered and washed with diethyl ether to yield the title compound as the dihydrochloride, analytically pure (2.63 g); mp 242°C.

NMR ($D_2O$): δ 1.5—2.0 (—$CH_2$—, 4 H, m), 2.8—3.3 (—$CH_2$—N, 4 H, m), 3.4—3.9 (=—$CH_2$—N, 4 H, m),

4.9—5.5 ( 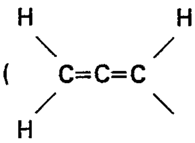 , 6 H, m);

IR (KBr):

1975 $cm^{-1}$ ( 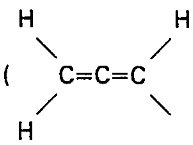 ).

Example 6

(E)-N[1],N[4]-2,3-Butadienyl-2-butene-1,4-diamine

A. (E)-N[1],N[4]-Tertiobutyloxycarbonyl-N[1],N[4]-2,3-butadienyl-2-butene-1,4-diamine

To a suspension of sodium hydride (0.426 g of a 55% suspension in oil washed 3 times with pentane, 9.82 mmol) and sodium iodide (catalytic amount) in anhydrous dimethylformamide (DMF, 9 ml) cooled to 0°C was added under nitrogen a solution of (E)-1,4-dibromo-2-butene (1.05 g, 4.91 mmol) in anhydrous DMF (4 ml). Then a solution of N-tertiobutyloxycarbonyl-2,3-butadienyl amine, prepared as in Example 3 (1.66 g) in anhydrous DMF was added dropwise over a period of 20 min. Stirring was continued for 30 min at 0°C. The reaction mixture was then quenched with water and extracted with diethylether (4 × 50 ml). The organic layers were pooled, washed with water, and then brine, and dried over $MgSO_4$. Concentration *in vacuo* afforded an oil (2.02 g) which was purified by flash chromatography (200 g of silica gel; eluant gradient of diethyl ether in petroleum ether 10 to 25%) to give the starting allenyl amine derivative (0.4 g) and the title compound (1.09 g) as an oil. The analytical sample was prepared by distillation with a "cold finger" apparatus (temp. oil bath: 125°C, 0.4 mm Hg).

NMR ($CDCl_3$): δ 1.45 ($C(CH_3)_3$, 18 H, s), 3.57—4.0 ($CH_2$—N, 8 H, m), 4.57—5.34

( 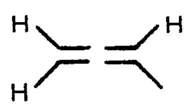 , 6 H, m);

5.35—5.60

( 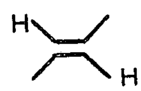 , 2 H, m);

IR ($CH_2Cl_2$): 1950 $cm^{-1}$ (=·=) 1680 $cm^{-1}$ (—$NCO_2$—);
MS (CI, $NH_3$) m/e $MH^+$: 391.

B. (E)-N[1],N[4]-2,3-Butadienyl-2-butene-1,4-diamine

To a solution of (E)-N[1],N[4]-tertiobutyloxycarbonyl-N[1],N[4]-2,3-butadienyl-2-butene-1,4-diamine, prepared as in Part A (0.994 g), in absolute ethanol (4 ml) was added a solution of anhydrous HCl (2 M) in anhydrous diethyl ether (12 ml). The mixture was left standing for 6 h at 25°C and then for 20 h at 4°C. The crystals which formed slowly were filtered and washed with anhydrous diethyl ether to yield the title compound analytically pure (0.555 g); mp 222°C.

NMR ($D_2O$): 3.4—4.0 (—$CH_2$—N, 4 H, m), 5.0—5.8

( 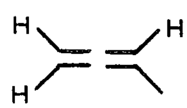 , 6 H, m);

6.0—6.3

( 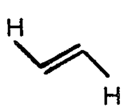 , 2 H, m);

IR (KBr): 1950 $cm^{-1}$ (allene).

Example 7

The ability of the compounds of Formula I to inhibit PAO *in vitro* can be demonstrated according to the following test procedure:

Measurements of kinetic constants:

Method:

Partially purified PAO from pig lover (forming 2.2 nmol $N^1$-acetylspermidin/mg protein/h from $N^1$,$N^{12}$-diacetylspermine) was preincubated with the inhibitor at 30°C in borate buffer at pH 9.0. The remaining enzyme activity was determined by measurement of the hydrogen peroxide formed [H. Snyder *et al., J. Pharm. Exptl. Therap., 63,* 386 (1968)] during the oxidation of $N^1$,$N^{12}$-diacetylspermine.

Results:

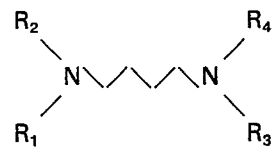

| Example | $R_1$ | $R_2$ | $R_3$ | $R_4$ | $K_i$(μM) | $\tau\frac{1}{2}$ (min) |
|---|---|---|---|---|---|---|
| 1 | 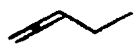 | H | H | H | 0.7 | 1 |
| 4 | 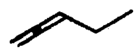 | H | H | $CH_3$ | 0.34 | 0.5 |
| 5 | 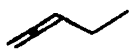 | H | H | 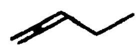 | 0.09 | 2.2 |

Example 8

The ability of the compounds of Formula II to inhibit PAO *ex vivo* can be demonstrated according to the following test procesure:

Measurement of PAO activities in mouse tissues:

Method:

PAO Activity was measured *ex vivo* in mouse tissue homogenates using $N^1$,$N^{12}$-diacetylspermine as substrate. $N^1$-Acetylspermidine was determined using a dansylation method [N. Seiler *et al., Biochim. Biophys. Acta, 615,* 480 (1980)].

Results:

$N^1$-2,3-Butadienyl-$N^4$-methyl-1,4-butanediamine, dihydrochloride (Example 4):

The dose-response in mice for PAO-inhibition was obtained at 4 h after i.p. injection. $ID_{50}$-values for liver, epididymis, spleen and kidney are between 0.05 and 0.1 mg/kg i.p. Complete inhibition in these organs can be achieved with a dose of 1 mg/kg i.p. The inhibition is irreversible; PAO is not reactivated *in vitro* by incubation with its substrate. For mouse brain-PAO the $ID_{50}$ is 5 mg/kg i.p.: inhibition is complete at 10 mg/kg.

$N^1$,$N^4$-2,3-Butadienyl-1,4-butanediamine, dihydrochloride (Example 5):

At a dose of 20 mg/kg i.p., this compound is equipotent to $N^1$-2,3-butadienyl-$N^4$-methyl-1,4-butanediamine, dihydrochloride (Example 4) in inhibiting PAO *in vivo.*

**Claims for the Contracting States: BE CH DE FR GB IT LI LU NL SE**

1. A compound of the formula:

$$H_2C{=}C{=}CHCH_2NHCH_2{-}Z{-}CH_2NHR$$

wherein:

Z is $-CH_2CH_2-$ or *trans*-$CH{=}CH-$,

R is H, $-CH_3$, $-CH_2CH_3$, $-(CH_2)_2CH_3$, $-(CH_2)_3NH_2$, $-(CH_2)_3NHCOCH_3$, $-CH_2CH{=}CH_2$, or $-CH_2CH{=}C{=}CH_2$, or a pharmaceutically acceptable acid addition salt thereof.

2. The compound as defined in Claim 1 which is $N^1$-2,3-butadienyl-$N^4$-methyl-1,4-butanediamine.

3. The compound as defined in Claim 1 which is $N^1$,$N^4$-2,3-butadienyl-1,4-butanediamine.

4. The compound as defined in Claim 1 which is $N^1$-2,3-butadienyl-$N^4$-aminopropyl-1,4-butanediamine.

5. The compound as defined in Claim 1 which is (E)-$N^1$,$N^4$-2,3-butadienyl-2-butene-1,4-diamine.

6. The compound as defined in Claim 1 which is (E)-$N^1$-2,3-butadienyl-$N^4$-acetylaminopropyl-2-butene-1,4-diamine.

7. A process for the preparation of a N-2,3-butadienyl-1,4-butanediamine derivative having the formula:

$$H_2C{=}C{=}CHCH_2NHCH_2{-}Z{-}CH_2NHR$$

wherein Z is $CH_2CH_2$ or *trans* CH=CH, R is H, $-CH_3$, $-CH_2CH_3$, $-(CH_2)_2CH_3$, $-(CH_2)_3NH_2$, $-(CH_2)_3NHCOCH_3$, $-CH_2CH{=}CH_2$ or $-CH_2CH{=}C{=}CH_2$ or a pharmaceutically acceptable salt thereof, which comprises reacting an N-protected 2,3-butadienylamine having the formula:

$$H_2C{=}C{=}CHCH_2NHB$$

wherein B represents an amino protecting group, with a protected N-alkyl or N-alkylene amine derivative having the formula:

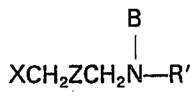

wherein B and Z are as described above, R′ is $-CH_3$, $-CH_2CH_3$, $-(CH_2)_2CH_3$, $-CH_2CH{=}CH_2$, $-CH_2CH{=}C{=}CH_2$ or $-CH_2CH_2CN$ and X is a leaving group, reacting said compounds in an organic solvent in the presence of a strong base at a temperature of from −30° to 100°C for a period of 10 minutes to 24 hours to form an amino protected N-2,3-butadienyl-1,4-butanediamine derivative having the formula:

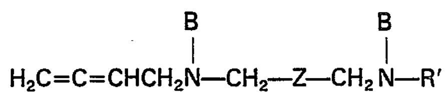

wherein B, Z and R′ are as defined above; and

(1) deprotecting said amino protected N-2,3-butadienyl-1,4-butanediamine derivative in a manner known *per se* to obtain the desired N-2,3-butadienyl-1,4-butanediamine derivatives having the formula:

$$H_2C{=}C{=}CHCH_2NHCH_2{-}Z{-}CH_2NHR'$$

wherein Z and R′ are as defined above, or

(2) when R′ is $-CH_2CH_2CN$, reducing said N-cyanoethyl derivative with lithium aluminium hydride to form the desired N-2,3-butadienyl-1,4-butanediamine derivative wherein R is $-(CH_2)_3NH_2$; or

(3) when R is $-(CH_2)_3NH_2$, forming the N-acetyl derivative thereof in a manner known *per se* to form the desired N-2,3-butadienyl-1,4-butanediamine derivative wherein R is $-(CH_2)_3NHCOCH_3$.

8. A process according to Claim 7 wherein the organic solvent is dimethylformamide, the strong base is sodium hydride, the temperature is from 0 to 25°C and the reaction time is from 20 minutes to $2\frac{1}{2}$ hours.

9. A process according to Claim 7 wherein the leaving group X is mesylate, tosylate, bromide or iodide.

10. A process according to Claim 7 wherein R is methyl and the N-2,3-butadienyl-1,4-butanediamine derivative is $N^1$-2,3-butadienyl-$N^4$-methyl-1,4-butanediamine.

11. A process according to Claim 7 wherein R is $-(CH_2)_3NH_2$ and the N-2,3-butadienyl-1,4-butanediamine derivative is $N^1$-2,3-butadienyl-$N^4$-aminopropyl-1,4-butanediamine.

12. A process according to Claim 7 wherein R is $-CH_2CH{=}C{=}CH_2$ and the N-2,3-butadienyl-1,4-butanediamine derivative is $N^1$,$N^4$-2,3-butadienyl-1,4-butanediamine.

13. A pharmaceutical composition comprising a compound of Claim 1 in admixture or otherwise associated with a pharmaceutically acceptable diluent or carrier.

14. A compound as defined in claim 1 for use as a medicine.

**Claims for the Contracting State: AT**

1. A process for the preparation of a N-2,3-butadienyl-1,4-butanediamine derivative having the formula:

$$H_2C{=}C{=}CHCH_2NHCH_2{-}Z{-}CH_2NHR$$

wherein Z is $CH_2CH_2$ or *trans* CH=CH, R is H, $-CH_3$, $-CH_2CH_3$, $-(CH_2)_2CH_3$, $-(CH_2)_3NH_2$, $-(CH_2)_3NHCOCH_3$, $-CH_2CH{=}CH_2$ or $-CH_2CH{=}C{=}CH_2$ or a pharmaceutically acceptable salt thereof, which comprises reacting an N-protected 2,3-butadienylamine having the formula:

$$H_2C{=}C{=}CHCH_2NHB$$

wherein B represents an amino protecting group, with a protected N-alkyl or N-alkylene amine derivative having the formula:

$$XCH_2ZCH_2N(B){-}R'$$

wherein B and Z are as described above, R' is $—CH_3$, $—CH_2CH_3$, $—(CH_2)_2CH_3$, $—CH_2CH{=}CH_2$, $—CH_2CH{=}C{=}CH_2$ or $—CH_2CH_2CN$ and X is a leaving group, reacting said compounds in an organic solvent in the presence of a strong base at a temperature of from −30°C to 100°C for a period of 10 minutes to 24 hours to form an amino protected N-2,3-butadienyl-1,4-butanediamine derivative having the formula:

$$H_2C{=}C{=}CHCH_2N(B){—}CH_2{—}Z{—}CH_2N(B){—}R'$$

wherein B, Z and R' are as defined above; and

(1) deprotecting said amino protected N-2,3-butadienyl-1,4-butanediamine derivative in a manner known *per se* to obtain the desired N-2,3-butadienyl-1,4-butanediamine derivatives having the formula:

$$H_2C{=}C{=}CHCH_2NHCH_2{—}Z{—}CH_2NHR'$$

wherein Z and R' are as defined above, or

(2) when R' is $—CH_2CH_2CN$, reducing said N-cyanoethyl derivative with lithium aluminium hydride to form the desired N-2,3-butadienyl-1,4-butanediamine derivative wherein R is $—(CH_2)_3NH_2$, or

(3) when R is $—(CH_2)_3NH_2$, forming the N-acetyl derivative thereof in a manner known *per se* to form the desired N-2,3-butadienyl-1,4-butanediamine derivative wherein R is $—(CH_2)_3NHCOCH_3$.

2. A process according to Claim 1 wherein the organic solvent is dimethylformamide, the strong base is sodium hydride, the temperature is from 0 to 25°C and the reaction time is from 20 minutes to 2½ hours.

3. A process according to Claim 1 wherein the leaving group X is mesylate, tosylate, bromide or iodide.

4. A process according to Claim 1 wherein R is methyl and the N-2,3-butadienyl-1,4-butanediamine derivative is $N^1$-2,3-butadienyl-$N^4$-methyl-1,4-butanediamine.

5. A process according to Claim 1 wherein R is $—(CH_2)_3NH_2$ and the N-2,3-butadienyl-1,4-butanediamine derivative is $N^1$-2,3-butadienyl-$N^4$-aminopropyl-1,4-butanediamine.

6. A process according to Claim 1 wherein R is $—CH_2CH{=}C{=}CH_2$ and the N-2,3-butadienyl-1,4-butanediamine derivative is $N^1$,$N^4$-2,3-butadienyl-1,4-butanediamine.

7. A process for preparing a pharmaceutical composition comprising a compound of Claim 1 which comprises mixing or otherwise associating such a compound with a pharmaceutically acceptable diluent or carrier.

**Patentansprüche für die Vertragsstaaten: BE CH DE FR GB IT LI LU NL SE**

1. Verbindung der Formel:

$$H_2C{=}C{=}CHCH_2NHCH_2{—}Z{—}CH_2NHR$$

wobei:

Z $—CH_2CH_2—$ oder *trans*-CH=CH— bedeutet

R H, $—CH_3$, $—CH_2CH_3$, $—(CH_2)_2CH_3$, $—(CH_2)_3NH_2$, $—(CH_2)_3NHCOCH_3$, $—CH_2CH{=}CH_2$ oder $—CH_2CH{=}C{=}CH_2$ bedeuten oder ein pharmazeutisch annehmbares Säureadditionssalz davon.

2. Verbindung nach Anspruch 1, welche $N^1$-2,3-Butadienyl-$N^4$-methyl-1,4-butandiamin ist.

3. Verbindung nach Anspruch 1, welche $N^1$,$N^4$-2,3-Butadienyl-1,4-butandiamin ist.

4. Verbindung nach Anspruch 1, welche $N^1$-2,3-butadienyl-$N^4$-aminopropyl-1,4-butandiamin ist.

5. Verbindung nach Anspruch 1, welche (E)-$N^1$,$N^4$-2,3-butadienyl-2-buten-1,4-diamin ist.

6. Verbindung nach Anspruch 1, welche (E)-$N^1$-2,3-butadienyl-$N^4$-acetylaminopropyl-2-buten-1,4-diamin ist.

7. Verfahren zur Herstellung eines N-2,3-Butadienyl-1,4-butandiamin-Derivates mit der Formel:

$$H_2C{=}C{=}CHCH_2NHCH_2{—}Z{—}CH_2NHR$$

wobei Z $CH_2CH_2$ oder *trans* CH=CH, R H, $—CH_3$, $—CH_2CH_3$, $—(CH_2)_2CH_3$, $—(CH_2)_3NH_2$, $—(CH_2)_3NHCOCH_3$, $—CH_2CH{=}CH_2$ oder $—CH_2CH{=}C{=}CH_2$ bedeuten oder ein pharmazeutisch annehmbares Salz davon, welches die Reaktion eines N-geschützten 2,3-Butadienylamin mit der Formel:

$$H_2C{=}C{=}CHCH_2NHB$$

wobei B eine Aminogruppe darstellt, mit einem geschützten N-Alkyl- oder N-Alkylenamin-Derivat mit der Formel:

B
|
$XCH_2ZCH_2N—R'$

wobei B und Z wie oben beschrieben sind, R' $—CH_3$, $—CH_2CH_3$, $—(CH_2)_2CH_3$, $—CH_2CH{=}CH_2$, $—CH_2CH{=}C{=}CH_2$ oder $—CH_2CH_2CN$ bedeutet und X eine Abgangsgruppe darstellt, beinhaltet, wobei die Reaktion besagter Verbindungen in einem organischen Lösungsmittel in Gegenwart einer starken Base bei einer Temperatur von −30°C bis 100°C für einen Zeitraum von 10 Minuten bis 24 Stunden durchgeführt wird, um ein Amino-geschütztes N-2,3-Butadienyl-1,4-butandiamin-Derivat zu bilden, welches die Formel:

B B
| |
$H_2C{=}C{=}CHCH_2N—CH_2—Z—CH_2\,N—R'$

hat, wobei B, Z und R' wie oben definiert sind und

(1) Entschützung besagten Amino-geschützten N-2,3-Butadienyl-1,4-butandiamin-Derivates in einer *per se* bekannten Weise durchgeführt wird, um die gewünschten N-2,3-Butadienyl-1,4-butandiamin-Derivate mit der Formel:

$$H_2C{=}C{=}CHCH_2NHCH_2—Z—CH_2NHR'$$

zu erhalten, wobei Z und R' wie oben definiert sind oder

(2) wenn R' $—CH_2CH_2CN$ bedeutet, die Reduktion des besagten N-Cyanoethyl-Derivates mit Lithiumaluminiumhydrid durchgeführt wird, um das gewünschte N-2,3-Butadienyl-1,4-butandiamin-Derivat zu bilden, wobei R $—(CH_2)_3NH_2$ bedeutet, oder

(3) wenn R $(—CH_2)_3NH_2$ bedeutet, die Bildung des N-Acetyl-Derivates davon in *per se* bekannter Weise durchgeführt wird, um das gewünschte N-2,3-Butadienyl-1,4-butandiamin-Derivat zu bilden, wobei R $—(CH_2)_3NHCOCH_3$ bedeutet.

8. Verfahren nach Anspruch 7, dadurch gekennzeichnet, daß das organische Lösungsmittel Dimethylformamid ist, die starke Base Natriumhydrid ist, die Temperatur zwischen 0 und 25°C beträgt und die Reaktionszeit zwischen 20 Minuten und 2½ Stunden liegt.

9. Verfahren nach Anspruch 7, dadurch gekennzeichnet, daß die Fluchtgruppe X Mesylat, Tosylat, Bromid oder Jodid ist.

10. Verfahren nach Anspruch 7, dadurch gekennzeichnet, daß R Methyl bedeutet und das N-2,3-Butadienyl-1,4-butandiamin-Derivat $N^1$-2,3-Butadienyl-$N^4$-methyl-1,4-butandiamin ist.

11. Verfahren nach Anspruch 7, dadurch gekennzeichnet, daß R $—(CH_2)_3NH_2$ bedeutet und das N-2,3-Butadienyl-1,4-butandiamin-Derivat $N^1$-2,3-Butadienyl-$N^4$-aminopropyl-1,4-butandiamin ist.

12. Verfahren nach Anspruch 7, dadurch gekennzeichnet, daß R $—CH_2CH{=}C{=}CH_2$ bedeutet und das N-2,3-Butadienyl-1,4-butandiamin-Derivat $N^1$,$N^4$-2,3-Butadienyl-1,4-butandiamin ist.

13. Pharmazeutische Zusammensetzung, die eine Verbindung nach Anspruch 1 in Mischung oder anders assoziiert mit einem pharmazeutisch annehmbaren Verdünnungsmittel oder Träger enthält.

14. Verbindung nach Aspruch 1 zur Verwendung als Medikament.

**Patentansprüche für den Vertragsstaat: AT**

1. Verfahren zur Herstellung eines N-2,3-Butadienyl-1,4-butandiamin-Derivates mit der Formel:

$$H_2C{=}C{=}CHCH_2NHCH_2—Z—CH_2NHR$$

wobei Z $CH_2CH_2$ oder *trans* $CH{=}CH$, R H, $—CH_3$, $—CH_2CH_3$, $—(CH_2)_2CH_3$, $—(CH_2)_3NH_2$, $—(CH_2)_3NHCOCH_3$, $—CH_2CH{=}CH_2$ oder $—CH_2CH{=}C{=}CH_2$ bedeutet oder ein pharmazeutisch annehmbares Salz davon, welches die Reaktion eines N-geschützten 2,3-Butadienylamin mit der Formel:

$$H_2C{=}C{=}CHCH_2NHB$$

wobei B eine Amino-Schutzgruppe darstellt, mit einem N-Alkyl- oder N-Alkylenamin-Derivat mit der Formel:

B
|
$XCH_2ZCH_2N—R'$

wobei B und Z wie oben beschrieben sind, R' $-CH_3$, $-CH_2CH_3$, $-(CH_2)_2CH_3$, $-CH_2CH{=}CH_2$, $-CH_2CH{=}C{=}CH_2$ oder $-CH_2CH_2CN$ und X eine Abgangsgruppe bedeuten, beinhaltet, wobei die Reaktion der besagten Verbindungen in einem organischen Lösungsmittel in Gegenwart einer starken Base bei einer Temperatur von −30°C bis 100°C für einen Zeitraum von 10 Minuten bis 24 Stunden durchgeführt wird, um ein Amino-geschütztes N-2,3-Butadienyl-1,4-butandiamin-Derivat der Formel:

$$H_2C{=}C{=}CHCH_2\overset{\overset{B}{|}}{N}-CH_2-Z-CH_2\overset{\overset{B}{|}}{N}-R'$$

zu bilden, wobei B, Z und R' wie oben definiert sind und

(1) Entschützung besagten Amino-geschützten N-2,3-Butadienyl-1,4-butandiamin-Derivates in einer *per se* bekannten Weise durchgeführt wird, um die gewünschten N-2,3-Butadienyl-1,4-butandiamin-Derivate mit der Formel:

$$H_2C{=}C{=}CHCH_2NHCH_2-Z-CH_2NHR'$$

zu erhalten, wobei Z und R' wie oben definiert sind, oder

(2) wenn R' $-CH_2CH_2CN$ bedeutet, die Reduktion des besagten N-Cyanoethyl-Derivates mit Lithiumaluminiumhydrid durchgeführt wird, um das gewünschte N-2,3-Butadienyl-1,4-butandiamin-Derivat zu bilden, wobei R $-(CH_2)_3NH_2$ bedeutet, oder

(3) wenn R $(-CH_2)_3NH_2$ bedeutet, die Bildung des N-Acetyl-Derivates davon in einer *per se* bekannten Weise, um das gewünschte N-2,3-Butadienyl-1,4-butandiamin-Derivat zu bilden, wobei R $-(CH_2)_3NHCOCH_3$ bedeutet.

2. Verfahren nach Anspruch 1, dadurch gekennzeichnet, daß das organische Lösungsmittel Dimethylformamid ist, die starke Base Natriumhydrid ist, die Temperatur zwischen 0 und 25°C beträgt und die Reaktionszeit zwischen 20 Minuten und 2½ Stunden liegt.

3. Verfahren nach Anspruch 1, dadurch gekennzeichnet, daß die Fluchtgruppe X Mesylat, Tosylat, Bromid oder Iodid bedeutet.

4. Verfahren nach Anspruch 1, dadurch gekennzeichnet, daß R Methyl bedeutet und das N-2,3-Butadienyl-1,4-butandiamin-Derivat $N^1$-2,3-Butadienyl-$N^4$-methyl-1,4-butandiamin ist.

5. Verfahren nach Anspruch 1, dadurch gekennzeichnet, daß R $-(CH_2)_3NH_2$ bedeutet und das N-2,3-Butadienyl-1,4-butandiamin-Derivat $N^1$-2,3-Butadienyl-$N^4$-aminopropyl-1,4-butandiamin ist.

6. Verfahren nach Anspruch 1, dadurch gekennzeichnet, daß R $-CH_2CH{=}C{=}CH_2$ bedeutet und das N-2,3-Butadienyl-1,4-butandiamin-Derivat $N^1,N^4$-2,3-Butadienyl-1,4-butandiamin ist.

7. Verfahren zur Herstellung einer pharmazeutischen Zusammensetzung, welche eine Verbindung nach Anspruch 1 enthält, welches Mischung oder andersartige Assoziation solch einer Verbindung mit einem pharmazeutisch annehmbaren Verdünnungsmittel oder Träger beinhaltet.

**Revendications pour les Etats contractants: BE CH DE FR GB IT LI LU NL SE**

1. Composé de formule:

$$H_2C{=}C{=}CHCH_2NHCH_2-Z-CH_2NHR$$

dans laquelle:

Z représente un groupe $-CH_2-CH_2$ ou trans-$CH{=}CH-$,

R représente H, $-CH_3$, $-CH_2CH_3$, $-(CH_2)_2CH_3$, $-(CH_2)_3NH_2$, $-(CH_2)_3NHCOCH_3$, $-CH_2CH{=}CH_2$, ou $-CH_2CH{=}C{=}CH_2$ ou un de ses sels d'addition d'acide pharmaceutiquement acceptable.

2. Composé tel que défini à la revendication 1, qui est la $N^1$-2,3-butadiènyl-$N^4$-méthyl-1,4-butanediamine.

3. Composé tel que défini à la revendication 1, qui est la $N^1,N^4$-2,3-butadiényl-1,4-butanediamine.

4. Composé tel que défini à la revendication 1, qui est la $N^1$-2,3-butadiényl-$N^4$-aminopropyl-1,4-butanediamine.

5. Composé tel que défini à la revendication 1, qui est la (E)-$N^1,N^4$-2,3-butadiényl-2-butène-1,4-diamine.

6. Composé tel que défini à la revendication 1, qui est la (E)-$N^1$-2,3-butadiényl-$N^4$-acétylaminopropyl-2-butène-1,4-diamine.

7. Procédé pour la préparation d'un dérivé de N-2,3-butadiényl-1,4-butanediamine, répondant à la formule:

$$H_2C{=}C{=}CHCH_2NHCH_2-Z-CH_2NHR$$

dans laquelle Z représente un groupe $CH_2CH_2$ ou trans $CH{=}CH$, R représente H ou un groupe $-CH_3$, $-CH_2CH_3$, $-(CH_2)_2CH_3$, $-(CH_2)_3NH_2$, $-(CH_2)_3NHCOCH_3$, $-CH_2CH{=}CH_2$ ou $-CH_2CH{=}C{=}CH_2$ ou un de ses

sels pharmaceutique acceptable, qui comprend la réaction d'une 2,3-butadiénylamine protégée sur l'azote, et répondant à la formule:

$$H_2C=C=CHCH_2NHB$$

dans laquelle B représente un groupe protecteur de fonction amine, avec un dérivé protégé de N-alkylamine ou de N-alkylèneamine répondant à la formule:

$$XCH_2ZCH_2\overset{\overset{B}{|}}{N}—R'$$

dans laquelle B et Z sont comme décrit ci-dessus, R' représente un groupe $—CH_3$, $—CH_2CH_3$, $—(CH_2)_2CH_3$, $—CH_2CH=CH_2$, $—CH_2CH=C=CH_2$ ou $—CH_2CH_2CN$ et X est un groupe qui part, la réaction desdits composés dans un solvant organique en présence d'une base forte à une température comprise entre −30° et 100°C en une période de 10 minutes à 24 heures pour former un dérivé de N-2,3-butadiényl-1,4-butanediamine à groupe amino protégé, répondant à la formule:

$$H_2C=C=CHCH_2\overset{\overset{B}{|}}{N}—CH_2—Z—CH_2\overset{\overset{B}{|}}{N}—R'$$

dans laquelle B, Z et R' sont tels que définis ci-dessus; et

(1) l'enlèvement du groupe protecteur, de façon connue en soi, du dérivé de N-2,3-butadiényl-1,4-butanediamine à groupe amino protégé, pour obtenir le dérivé voulu de N-2,3-butadiényl-1,4-butanediamine répondant à la formule:

$$H_2C=C=CHCH_2NHCH_2—Z—CH_2NHR'$$

dans laquelle Z et R' sont tels que définis ci-dessus, ou

(2) quand R' représente un groupe $—CH_2CH_2CN$, la réduction de ce dérivé de N-cyanoéthyle par de l'hydrure de lithium et d'aluminium pour former le dérivé voulu de N-2,3-butadiényl-1,4-butanediamine dans lequel R représente un groupe $—(CH_2)_3NH_2$, ou

(3) quand R représente un groupe $—(CH_2)_3NH_2$, la formation du dérivé N-acétylé de ce composé, d'une manière connue en soi, pour former le dérivé voulu de N-2,3-butadiényl-1,4-butanediamine dans lequel R représente $—(CH_2)_3NHCOCH_3$.

8. Procédé selon la revendication 7, dans lequel le solvant organique est le diméthylformamide, la base forte est l'hydrure de sodium, la température se situe entre 0 et 25°C et le temps de réaction se situe entre 20 minutes et 2 heures $\frac{1}{2}$.

9. Procédé selon la revendication 7, dans lequel le groupe qui part est un groupe mésylate, tosylate, bromure ou iodure.

10. Procédé selon la revendication 7, dans lequel R représente un groupe méthyle, et le dérivé de N-2,3-butadiényl-1,4-butanediamine est la $N^1$-2,3-butadiényl-$N^4$-méthyl-1,4-butanediamine.

11. Procédé selon la revendication 7, dans lequel R représente un groupe $—(CH_2)_3NH_2$, et le dérivé de N-2,3-butadiényl-1,4-butanediamine est la $N^1$-2,3-butadiényl-$N^4$-aminopropyl-1,4-butanediamine.

12. Procédé selon la revendication 7, dans lequel R représente un groupe $—CH_2CH=C=CH_2$, et le dérivé de N-2,3-butadiényl-1,4-butanediamine est la $N^1$,$N^4$-2,3-butadiényl-1,4-butanediamine.

13. Composition pharmaceutique, comprenant un composé selon la revendication 1, en mélange ou en association d'une autre façon avec un diluant, véhicule ou excipient ou support pharmaceutiquement acceptable.

14. Composé tel que défini à la revendication 1, destiné à servir de médicament.

**Revendications pour l'Etat contractant: AT**

1. Procédé pour la préparation d'un dérivé de N-2,3-butadiényl-1,4-butanediamine, répondant à la formule:

$$H_2C=C=CHCH_2NHCH_2—Z—CH_2NHR$$

dans laquelle Z représente un groupe $CH_2CH_2$ ou trans $CH=CH$, R représente H ou un groupe $—CH_3$, $—CH_2CH_3$, $—(CH_2)_2CH_3$, $—(CH_2)_3NH_2$, $—(CH_2)_3NHCOCH_3$, $—CH_2CH=CH_2$ ou $—CH_2CH=C=CH_2$ ou un de ses sels pharmaceutiquement acceptable, qui comprend la réaction d'une 2,3-butadiénylamine protégée sur l'azote, et répondant à la formule:

$$H_2C=C=CHCH_2NHB$$

dans laquelle B représente un groupe protecteur de fonction amine, avec un dérivé protégé de N-alkylamine ou de N-alkylèneamine, répondant à la formule:

$$XCH_2ZCH_2\overset{\overset{B}{|}}{N}—R'$$

dans laquelle B et Z sont comme décrit ci-dessus, R' représente un groupe $—CH_3$, $—CH_2CH_3$, $—(CH_2)_2CH_3$, $—CH_2CH{=}CH_2$, $—CH_2CH{=}C{=}CH_2$ ou $—CH_2CH_2CN$ et X est un groupe qui part, la réaction desdits composés dans un solvant organique en présence d'une base forte à une température comprise entre −30°C et 100°C en une période de 10 minutes à 24 heures pour former un dérivé de N-2,3-butadiényl-1,4-butanediamine à groupe amino protégé, répondant à la formule:

$$H_2C{=}C{=}CHCH_2\overset{\overset{B}{|}}{N}—CH_2—Z—CH_2\overset{\overset{B}{|}}{N}—R'$$

dans laquelle B, Z et R' sont tels que définis ci-dessus; et

(1) l'enlèvement du groupe protecteur, de façon connue en soi, du dérivé de N-2,3-butadiényl-1,4-butanediamine à groupe amino protégé, pour obtenir le dérivé voulu de N-2,3-butadiényl-1,4-butanediamine répondant à la formule:

$$H_2C{=}C{=}CHCH_2NHCH_2—Z—CH_2NHR'$$

dans laquelle Z et R' sont tels que définis ci-dessus, ou

(2) quand R' représente un groupe $—CH_2—CH_2CN$, la réduction de ce dérivé de N-cyanoéthyle par de l'hydrure de lithium et d'aluminium pour former le dérivé voulu de N-2,3-butadiényl-1,4-butanediamine dans lequel R représente un groupe $—(CH_2)_3NH_2$, ou

(3) quand R représente un groupe $—(CH_2)_3NH_2$, la formation du dérivé N-acétylé de ce composé, d'une manière connue en soi, pour former le dérivé voulu de N-2,3-butadiényl-1,4-butanediamine dans lequel R représente $—(CH_2)_3NHCOCH_3$.

2. Procédé selon la revendication 1, dans lequel le solvant organique est le diméthylformamide, la base forte est l'hydrure de sodium, la température se situe entre 0 et 25°C et le temps de réaction se situe entre 20 minutes et 2 heures ½.

3. Procédé selon la revendication 1, dans lequel le groupe qui part est un groupe mésylate, tosylate, bromure ou iodure.

4. Procédé selon la revendication 1, dans lequel R représente un groupe méthyle, et le dérivé de N-2,3-butadiényl-1,4-butanediamine est la $N^1$-2,3-butadiényl-$N^4$-méthyl-1,4-butanediamine.

5. Procédé selon la revendication 1, dans lequel R représente un groupe $—(CH_2)_3NH_2$, et le dérivé de N-2,3-butadiényl-1,4-butanediamine est la $N^1$-2,3-butadiényl-$N^4$-aminopropyl-1,4-butanediamine.

6. Procédé selon la revendication 1, dans lequel R représente un groupe $—CH_2CH{=}CH{=}CH_2$, et le dérivé de N-2,3-butadiényl-1,4-butanediamine est la $N^1$,$N^4$-2,3-butadiényl-1,4-butanediamine.

7. Procédé pour préparer une composition pharmaceutique comprenant un composé selon la revendication 1, qui comprend le mélangeage ou l'association d'une autre manière d'un tel composé avec un diluant, véhicule, support ou excipient pharmaceutiquement acceptable.